# EUROPEAN PATENT SPECIFICATION

(11) **EP 0 868 914 B1**
(45) Date of publication and mention of the grant of the patent: **18.12.2002**
(21) Application number: 97302242.9
(22) Date of filing: 01.04.1997
(51) Int. Cl.: A61K 31/35, A61K 31/70, A61K 31/78, A61P 1/00

(54) **USE OF A FLAVONOID-CONTAINING EXTRACT OF THE PLANT EUPHORBIA PROSTRATA IN THE MANUFACTURE OF A MEDICAMENT FOR THE TREATMENT OF AN ANORECTIC OR COLONIC DISEASE**
VERWENDUNG EINES FLAVONOID ENTHALTENDEN EXTRAKTES DER PLANZE EUPHORBIA PROSTRATA ZUR HERSTELLUNG EINES MEDIKAMENTES ZUR BEHANDLUNG VON ANORAKTALEN ODER KOLONBESCHWERDEN
UTILISATION D'UN EXTRAIT CONTENANT DES FLAVONOIDES DE LA PLANT EUPHORBIA PROSTRATA POUR LA FABRICATION D'UN MEDICAMENT POUR LE TRAITMENT DES DESORDRES COLONIQUES ET ANORECTAUX

(43) Date of publication of application: 07.10.1998
(73) Proprietor: PANACEA BIOTEC LIMITED, New Dehli 110001 (IN); UNIVERSITY INSTITUTE OF PHARMACEUTICAL SCIENCES, Chandigarh 160014, Punjab (IN)
(72) Inventor: Singh, Amarjit, New Delhi-110001 (IN); Jain, Rajesh, New Delhi-110001 (IN); Singla, Anil Kumar, Chandigarh 160014, Punjab (IN)
(74) Representative: Howard, Paul Nicholas

(56) References cited:
- EP-A- 0 454 127
- DATABASE WPI Week 9346 Derwent Publications Ltd., London, GB; AN 93-365154 XP002039563 & JP 05 271 088 A (RUIBOSUTI JAPAN KK) , 19 October 1993

## Description

The invention relates to the use of a flavonoid - containing extract of the plant Euphorbia prostrata in the manufacture of a composition for the treatment of anorectal and colonic diseases (including hemorrhoids). The composition possess properties to control inflammation, prevent capillary bleeding and fragility in mammals particularly human beings.

### BACKGROUND OF THE INVENTION

Anorectal and colonic diseases are usually characterised by inflammation of the infected region. Other common symptoms are those associated with inflammation, like heat, itching, redness, pain and swelling. A common characteristic of such anorectal and colonic diseases is the appearance of fissures, cracks, fistulas and abscesses.

Among the various anorectal and colonic diseases, hemorrhoids occupy a prominent position and have been the subject of numerous clinical studies. Hemorrhoidal disease is characterised by bleeding, without any pain. Fresh blood spots occur immediately, on defecation. However, pain occurs when the hemorrhoids are secondarily infected, or complicated by thrombosis and anal fissures. Hemorrhoids is characterised by episodes of acute hemorrhoid attacks, with bleeding, pain and prolapse of hemorrhoidal mass.

Thus, an effective treatment of acute hemorrhoidal attacks should not only provide relief as early as 2-3 days, after initiation of the treatment, but also reduce the recurrence of such attacks.

There exist several procedures for the treatment of hemorrhoids. Patents have been granted in respect of surgical dressings (European patent No.8803398) and surgical devices (European patent No.0095142). A patent, (United States Patent No.4621635) has been granted for the use of lasers in the treatment of hemorrhoids. The techniques of cryopharmacotherapy and electrochemical techniques for treatment of hemorrhoids have also been patented vide European patent No.0091405 and European Patent No.0116688, respectively. However, the biggest drawbacks of the above, are the involvement of medical experts beyond mere prescription of medicines and probable hospitalisation. Also, some of them are physically and/or psychologically unpleasant in application.

Several patents (Nos.4160148, 4508728, 4797392, 4518583 and 5234914) have been granted in the United States of America in respect of compositions containing certain wound healing agents to provide symptomatic relief, by promoting tissue repair, reducing inflammation and encouraging wound healing. Some of them like United States Patent Nos.4518583 and 5234914 contain antimicrobial agents. These compositions, however, only relieve symptoms associated with inflammation, like heat, itching, redness, pain and swelling.

A number of compositions for the treatment of anorectal diseases (including hemorrhoids) are based on the anaesthetic and vasoconstrictive properties of the constituents, but these provide only temporary symptomatic relief.

Patents in the United States of America (United States Patents Nos.4613498, 4626433, 5166132, 5219880, 5234914 and 4797392) and Europe (European Patents Nos.8518086 and 0513442) have been granted in respect of compositions with varying constituents, for topical application in the form of suitable and acceptable pharmaceutical carriers, such as salts, salves, ointments etc., with organic, inorganic and biological active agents. However, these compositions provide only temporary relief and are limited to local application and cannot be used for systemic use or oral administration.

A topical treatment for hemorrhoidal pain and for spasms of the sphintcters and muscles located in the GI tract is disclosed in a granted patent (US patent 595753) which includes amino acid L-arginine in a pharmaceutically acceptable carrier.Another US patent (5591436) has been granted for a composition for dietary supplement for the treatment of hemorrhoids. The composition comprisis 60% to 95% Indian Barberry by weight; 4.8% to 38% Nagkesar by weight; and 0.2% to 2% Margosa tree leaves by weight.

Another US patent (5562906) discloses the use of bark or berries of the species *Xanthoxylum clavaherculis* L and *Xanthoxylum americanum* Hill, both of the yellow wood tree family, both containing the compound Xanthoxylum are employed for the treatment of hemorrhoids and other membrane and capillary disorders of the veins and arteries. Improved strength and flexibility of the veins, arteries and their constituent structures is obtained.

There is available in the market, pharmaceutical compositions containing diosmin and a combination of diosmin and hesperidin for the treatment of hemorrhoids. However, the concentration of diosmin and hesperidin in such compositions is much higher than the concentrations of flavonoids of the present invention. Besides such diosmin and hesperidin are obtained from synthetic sources.

The inventors have researched, and as a result of the expenditure of time and mental faculties have found that the flavonoid composition of the present invention exhibits surprisingly significant enhanced pharmacological and therapeutic response at much lower dosage levels in comparison to existing compositions employing flavonoids.

The present invention provides a Pharmaceutical composition which is safe and painless and has long term effectiveness.

### SUMMARY OF THE INVENTION

Use of a flavenoid-containing extract of the plant Euphorbia prostrata in the manufacture of a composition for treating anorectal diseases including hemorrhoids and colonic diseases with long term effectiveness and low prolapse rates. The treatment includes applying to the hemorrhoids and anorectal tissues an effective amount of composition including a pharmaceutically acceptable carrier and a mixture of flavonoids. The treatment also includes adminstration by oral route and I or parenteral route an effective amount of composition including a pharmaceutically acceptable carrier and mixture of flavonoids

### DETAILED DESCRIPTION OF THE PREFERRED EMBODIMENTS

This invention provides the use of compositions which are water soluble and can be uniformly distributed in the affected region. It reduces inflammation, and soothes the feeling of itching and burning associated with it. The invention also provides relief from pain, which characterises hemorrhoids. Growth of microorganisms is also prevented by the composition when administered in any manner. The invention is useful in the treatment of lesions, other than hemorrhoids in the anorectal area and can be formulated in several types of Dosage Forms. There are no side effects from the use of the composition in human beings. Further, the treatment is not physically or psychologically unpleasant in its application. The plant *Euphorbia prostrata* (Family: Euphorbiaceae) was identified as being relevant in the study of anorectal and colonic diseases, including hemorrhoids. *Euphorbia prostrata* is well known to the Indian traditional medicine in the use of treatment for asthma, bloody dysentery and sores. Five new compounds were discovered and identified by the inventors in *Euphorbia prostrata* (Fig. 1) namely luteolin, 6-methoxy-quercetin-glycoside, Querceitin, and glycosides of luteolin and apigenin. *Euphorbia prostrata* contains 1-2.5% of total flavonoids. Out of which apigenin glycoside is 0.8-1.4%, luteolin glycoside is 0.2-0.5%, 6-methoxy-quercetin glycoside is 0.2%, quercetin and luteolin is 0.1%.

These flavonoidal constituents are reported to have anti-inflammatory properties. These compounds were extracted from *Euphorbia prostrata* for testing and combined in a proportion, which is new and has not been reported before. The compounds in such proportion were standardised to pharmaceutically acceptable specifications in order to ensure reproducibility from batch to batch. The result is the standardized extract of *Euphorbia prostrata*, which is the main active agent in the improved anorectal composition. Another unique feature of this extract of *Euphorbia prostrata* is, that it is prepared in such a manner that the resulting composition is water soluble. Pharmaceutical dosage carriers used in the present invention are capsules, tablets, ointments, creams, gels, foams, aerosols, sprays, and the like.

This invention further provides a process for the manufacture of a an extract which comprises drying the plant *Euphorbia Prostrata* under controlled conditions of temperature and humidity, making a powder from the said dried plant, extracting the dry coarse powder to form an extract, filtering and distilling the said extract repetitively, dehydrating the said extract, drying the extract in a vacuum to produce the desired pharmaceutical extract capable of being used along with a suitable carrier/base.

In a preferred embodiment the said dry coarse powder is extracted with aqueous alcohol.

In another preferred embodiment the extract is filtered and extracted with ethyl acetate.

In another preferred embodiment the dehydration is carried out over anhydrous sodium sulphate.

Other plants containing apigenin glycosides and luteolin glycosides are *Ixora arborea* (Rubiaceae), *Bommervia hispida* (Pteridaceae), *Adenocalymma alliaceum* (Bignomiaceae), *Thalictrum thunbergii* (Renunculaceae), *Perilla frutescens* (Labiateae) *Matricaria chamomilla* (Compositae), *Thymus membranaceous* (Labiateae), *Digitalis Ianata* (Scrophulariaceae), *Cuminum cyminum* (Umbelliferae), *Petroselinum, Euphorbia minuta, E.Serpeus-microfolia,* E.granulata. *Chrysenthemum indicum* and *Matricaria chamomilla* (Compositae) contain both apigenin and luteolin.

Glycosides of quercetin has been reported from different species of *Euphorbia (E. verrueosa, E. platiphyllos, E.discolor, E. dulcis, E. helioscopia, E. thymifolia* etc).

The pharmaceutical composition containing the standardised extract of *Euphorbia prostrata* as the active ingredient contains 35-62% flavonoids. Out of which apigenin glycoside is 30-45%, luteolin glycoside is 3-9%, 6-methoxy quercetin glycoside is 1-6%, quercetin and luteolin is 1-2%. The Extract also contains tannins (5%), resins and gums (10-15%), besides pigments, sterols, triterpenoids etc.,

The pharmaceutical composition of the present invention may also contain the active agents from other plants and/or from different pharmacological groups such as local anesthetics, vasoconstrictors, protectants, counterirritants, astringents, keratolytics and anticholinergics.

Preferably, it would be beneficial to include other wound healing and antimicrobial agents which will result in the improvement of the effectiveness of the composition.

The local anesthetics and/or their salts, include but are not limited to such as benzocaine, diperodon, pramoxine, camphor, dibucaine, phenol, tetrtacaine, lignocaine and phenacaine. The amount of such anesthetics could vary between 0.25% and 25% by weight.

The vasoconstrictors include but are not limited to ephedrine, phenylephrine, phenylephrine and/or their salts. The amount of such vasoconstrictors may vary between 0.005% and 1.5% by weight.

The protectants include but are not limited to aluminium hydroxide gel, calamine, cocoa butter, cod or shark liver oil, glycerin in aqueous solution, kaolin, lanolin, mineral oil, starch, white petrolatum, wool alcohol, zinc oxide, vegetable or castor oil, polyethylene glycol and propylene glycol. The amount of such protectants may vary between 5.0% and 88.0% by weight.

The counterirritant includes but is not limited to menthol in aqueous solution. The amount of such counterirritant may vary between 0.25 - 2.5% by weight.

The astringents include but are not limited to calamine, zinc oxide, hamamelis water, bismuthresorcinol compound, bismuth subgallate, peruvian balsam, aluminium chlorhydroxy allantoinate, tannic acid and tannins. The amount of such astringents may vary between 0.2% to 60.0% by weight. The tannins additionally may be derived from plants such as *Butea monosperma* (Family: Leguminosae) *Butea parviflora* and *Butea frondoza*.

The wound healing agents include but are not limited to vitamin A and vitamin D in an amount by weight of between 0.005% to 0.04%. Also peruvian balsam can be included by weight in an amount of between about 0.5% to 2.5%. Also cod liver oil can be included by weight in an amount between 1.0% to 6.0%. Also live yeast cell derivative can be included in an amount of between 2-50,000 units per gram.

The antimicrobial agents include but are not limited to benzethonium chloride, benzalkonium chloride, boric acid, 8-quinolinol benzoate, secondary amyltricresols, cetylpyridinium chloride, phenol, menthol, chlorothymol, camphor and 8-hydroxyquinoline sulfate. The amount of such antimicrobial agents vary between 0.02% and 40.0% by weight.

The keratolytics include but are not limited to aluminium chlorhydroxy allantoinate and resorcinol. The amount of such keratolytics may vary between 0.2% and 3.5% by weight.

The anticholinergics include but are not limited to atropine or other solanaceous type alkaloids, either alone or in combination. The amount of such anticholinergics may vary between 0.02% and 0.1% by weight.

The pharmaceutical compositions of the present invention can be dissolved or dispersed in an appropriate base, which can be suppositories, ointments, foams, sprays, medicated pads, capsules and tablets.

The capsules contain 5-50mg of the standardized extract of *Euphorbia prostrata*, preferably 9-15 mg alongwith pharmaceutical excipients. Similarly, tablets may be prepared by dispersing 5-50mg of the standardized extract of *Euphorbia prostrata*, preferably 9-15 mg alongwith pharmaceutical excipients. The tablets may be coated or uncoated.

The ointment may contain 0.1-10% w/w of the standardized extract of *Euphorbia prostrata*, preferably 0.2-5%. The capsule may be taken, subject to a maximum of 100mg per day, alongwith topical application containing the same extract, as and when required.

The granules in ready dispersible and effervescent form may be prepared by using excipients sucrose, mannitol, sodium bicarbonate, citric acid, sodium chloride etc.,

The cream may be prepared by emulsifying the aqueous phase, containing the active agent (0.1-10%, w/w preferably 0.2-5%), along with a suitable oleagenous phase, such that it results in a water soluble cream.

Other alternatives can be prepared by formulating the Standardized Extract in 0.1-10% w/w as hydrophilic ointment (USP) absorption base, or water soluble bases, such as PEG ointment, (USNF) or as water absorbing bases such as hydrophilic petrolatum USP, lanolin USP or in hydrocarbon bases, such as white petrolatum USP.

The suppository compositions may contain either hydrophobic or hydrophilic base and can include cocoa butter, glycerinated gelatin, hydrogenated vegetable oils, mixtures of polyethylene glycols of various molecular weights, polyoxyethylene sorbitan fatty acid esters and polyethylene stearates, polyvinyl alcohol, polyvinyl pyrrolidone, polyacrylamide, chemically modified starch or a combination of these materials.

The foam and spray bases may contain one or more of aqueous and non aqueous solvents, propellants, surfectants, suspending and stabilising agents.

The medicated pads may contain one or more of the following: Water, glycerin, propylene glycol, alcohol and Hamamelis water.

### EVALUATION OF PHARMACOLOGICAL ACTIVITY OF THE STANDARDIZED EXTRACT

### ORAL ANTI INFLAMMATORY ACTIVITY (AIA):

Studies with the standardized extract of *Euphorbia prostrata*, when administered orally showed an inhibition of both carrageenan-induced oedema at t= 180 min (b = 0.019, r = 0.965, p <0.001) with ED₅₀ value of 5.98 mg/kg (95% c. I = 11. 71 - 23.68) and histamine-induced oedema (b = 0.019, r = 0.860.p<0.001) with ED₅₀ value = 16.37 mg/kg (95% CL = 0.01-32.73).

Carrageenan-induced oedema is a biphasic event, the early hypermia being due to the release of histamine and serotonin and the delayed oedema due to the release of bradykinin and prostaglandins, The findings shown in Table 1, indicate that the orally administered standardized extract (200mg/kg) is effective against both phases of inflammation, inhibiting 76% of oedema and equivalent to 74% inhibition by 1mg/kg of indomethacin (i.p.).

The results have been further supported by the experiments carried out in histamine and bradykinin induced oedema models. The data shown in Table 2 indicates 77% and 56% inhibition of oedema at +60 and +120 min in the histamine-induced oedema model, while in the bradykinin-induced model the effect was significant (p<0.001) only at + 180 min.

**Table 1**

| Modification of Carrageenan-induced Pedal Oedema in Rats following oral Administration of the extract and Intraperitoneal treatment with Indomethacin. | | | | | | |
|---|---|---|---|---|---|---|
| | **Dose mg/Kg** | **N** | **Oedema + SEM (%)** | | | |
| | | | **+30** | **+60** | **+120** | **+180 min.** |
| The Standardized | 50 | 6 | 8.5±2.8 | 23.9±1.7 | 37.7±5.7 | 48.5±5.0** |
| Extract | 100 | 6 | 3.1±1.4* | 9.2±1.8*** | 25.4±2.0*** | 36.2±0.9*** |
| | 200 | 6 | 0.7±4.5** | 1.4±4.7*** | 13.6±7.5*** | 15.0±9.9*** |
| | | | | | | |
| Indomethacin | 0.5 | 6 | 13.6±4.0 | 18.9±4.0 | 28.8±4.2 | ND |
| | 1.0 | 6 | 9.3±2.3 | 12.3±2.2 | 16.4±1.6 | ND |
| | 2.0 | 6 | 8.2±1.5 | 12.8±1.8 | 11..8±1.8 | ND |
| | | | | | | |
| Control | - | 22 | 13.0±2.7 | 23.3±3.0 | 51.5±4.6 | 62.7±2.9 |

| | | | | | | |
|---|---|---|---|---|---|---|
| Significance relative to the respective control. Group data: * P<0.01; | | | | | | |
| ** P<0.05; | | | | | | |
| *** P<0.001 ND = Not Determined | | | | | | |

**Table 2**

| Effect of the extract (100 mg/Kg, orally) on Histamine and Bradykinin induced Oedema | | | | | |
|---|---|---|---|---|---|
| **Treatment** | **N** | **Oedema + SEM (%)** | | | |
| | | **+30** | **+60** | **+120** | **+180 min.** |
| Histamine | 6 | 19.0±0.3 | 43.8±0.3 | 53.3±0.6 | 55.2±0.9 |
| | | | | | |
| Histamine+Extract | 6 | 6.1±1.0* | 9.9±1.1* | 22.1±1.2* | 35.9±0.6 |
| | | | | | |
| 3radykinin | 6 | 14.3±0.6 | 19.3±0.8 | 29.4±0.4 | 47.0±0.6 |
| | | | | | |
| Bradykinin+Extract | 6 | 13.1±0.7 | 14.0±1.3 | 29.0±0.9 | 36.5±0.4 |

| | | | | | |
|---|---|---|---|---|---|
| Significance relative to the respective control group data * p<0.001. | | | | | |

### TOPICAL ANTIINFLAMMATORY ACTIVITY:

### Carrageenan-induced Pedal Oedema Test:

Dose dependent antiinflammatory response was observed with the standardized extract (b=0.0287, r=0.991, p<0.001 with ED₅₀ value = 9.6 % (95% CL =8.56-10.72) as well as with the mixture of Apigenin glycoside & Luteolin glycoside (4:1) (b=0.0169, r=0.989,p< 0.001) with ED₅₀ value = 1.12% (95% CL = 0.045 - 2.195) when calculated with + 180 min data on topical application, in the carrageen-induced foot paw oedema in mice.

It is thus concluded that the standardized extract is an antiinflammatory agent against two models of acute inflammatory reaction but appears to be relatively less potent than Indomethacin given by a different route (i.p.).

**Table 3**

| Modification of Carrageenan-induced Mice Paw Oedema following Topical Application of the Standardized Extract and Indomethacin. | | | | | | | | |
|---|---|---|---|---|---|---|---|---|
| Treatment | Dose (%) | N | +60 | +120 | +180 | +240 | +360 | +480m |
| The Standardized Extract | 0.5 | 6 | - | 2.0 | 4.8 | 5.4** | 1.9 | 2.3 |
| | 1.0 | 6 | 12.1** | 16.0* | 17.7 | 10.9* | 3.8 | 2.3 |
| | 2.0 | 6 | 9.1 | 16.0* | 24.2* | 18.2* | 7.7 | 4.6 |
| | 4.0 | 6 | 24.2** | 26.0* | 37.1*** | 29.1* | 7.7** | 6.9 |
| | 0.25 | 6 | 10.6** | 9.0* | 12.3*** | 8.9* | 5.2* | 7.9 |
| | 0.50 | 6 | 27.2** | 26.0* | 29.0* | 14.5* | 11.5* | 9.3 |
| | 1.00 | 6 | 30.3 | 28.0* | 46.8*** | 21.2* | 11.5* | 13.9 |
| Indomethacin | 1.00 | 6 | 18.2** | 24.0** | 29.0** | 21.2** | 9.6** | 4.6 |
| | 2.00 | 6 | 24.2* | 26.0* | 41.9*** | 36.4* | 11.5* | 10.6 |

| | | | | | | | | |
|---|---|---|---|---|---|---|---|---|
| Significance releative to the respective control group data: ** p<0.01 | | | | | | | | |
| ** p<0.05 | | | | | | | | |
| *** p<0.001 | | | | | | | | |

The duration of action of topical preparation was determined by studying the effects of different concentrations of the drug on oedema over a period of 8 hr. As evident from the Table 3, the peak inhibition was observed after 3-4 hours of carrageenan injection begining at 2 hr for the extract at lower concentrations (0.5-2.0%) and 1 hr. at 4 % concentration. A similar kinetic response was observed for the mixture of Apigenin glycoside & luteolin glycoside (4:1)(0.2-1%) and Indomethacin (1-2%). The maximal effect of the mixture of apigenin glycoside and luteolin glycoside preparations observed at 4% concentration (37.1% inhibition) and 1 % concentration (46.8% inhibtion), respectively, was comparable to that of indomethacin at 2 percent concentration (41.9% inhibition).

The topical effects of indomethacin were comparable with published results of mice carragenan-induced paw oedema (Schrier, D.J., Moniot, S., Gluckman, M.I. and Gilberston, R.B., J. Pharm. Pharmacol., 39, 57, 1989).

The mixture of apigenin glycoside & luteolin glycoside was found to possess significant AIA on oral administration with ED₅₀ value=5.98 mg/kg (1.12%) to carageenan-induced paw oedema in rats which is comparable to ED₅₀ value=1.11% obtained on evaluation of topical AIA data of same fraction, suggesting that this mixture of compounds has both topical as well as oral AIA. The oral absorption of drug as a result of preening may also be considered in topical effect. However, percutaneous absorption of apigenin glycoside has also been reported. When compared to indomethacin this fraction demonstrated a greater antiinflammatory action and slightly higher inhibition of the later phases of oedema. This is attributed to the activity of luteolin glycoside present in the fraction which is reported to decrease swelling in the later phase of oedematus reaction while apigenin glycoside acts in the earlier phase. Hence it is concluded that the standardized extract and the mixture of apigenin glycoside & luteolin glycoside have signifincant AIA. Further, comparable ED₅₀ values of topical and oral administration of the standardized extract suggest that the topical effect appears to be independent of systemic absorption.

### Croton Oil Ear Test:

The test used was described in published literature, where apigenin 7-glucoside was used as a reference sample. Luteolin glycoside (300µg/ear) significantly inhibited the croton-oil induced (72%) and showed a dose dependent inhibition of the oedematous response to corton oil. The activity of the compound is comparable to that of apigenin 7-glucoside. Since the standardized extract also contain similar glycosides, hence also active, but to a lesser extent than the mixture of apigenin glycoside & luteolin glycoside. From this data it can be concluded that these glycosides possess an AIA similar to that already described for other chemically related glycosides.

This comparative study of the standardized extract, apigenin glycoside, luteolin glycoside, and the mixture of apigenin glycoside & luteolin glycoside was confirmed independently by a Pharmacology Institute in Italy on the instruction of the inventors. The data on croton oil ear test in mice was found in agreement with data obtained in our laboratory using carrageenan model; any difference in the responses was attributed to the different administration routes.

Some of the results obtained by Italian Institute are reproduced below (Table 4). Indomethacin and hydrocortisone were used as the reference drugs.

**Table 4 :**

| Anti inflammatory activity of the products tested. | | | |
|---|---|---|---|
| | ID₅₀ | Activity Ratio^{a} | |
| Products | µg/ear | µmol/ear | on Molar basis |
| Indomethacin | 46.1 | 0.126 | 1.00 |
| Hydrocortisone | 3.3 | 0.009 | 14.00 |
| Apigenin glycoside* | 29.8 | 0.110 | 1.18 |
| Luteolin glycoside* | 38.4 | 0.135 | 0.96 |
| Quercetin* | 60.3 | 0.200 | 0.65 |
| Apigenin -7-glucoside | 175 | 0.404 | 0.32 |
| Rutin | 282 | 0.462 | 0.28 |

| | | | |
|---|---|---|---|
| ^{a} Indomethacin = 1 | | | |
| * Main constiuents of the standardized extract. | | | |

Apigenin glycoside and luteolin glycoside appear to be the most active compounds among the compounds tested and are similar in potency to indomethacin, whereas other glycosides are less active. The potency of aglycones has been shown to be strictly propotional to their polarity (data not shown) suggesting that the percutaneous absorption may be an activity-limiting factor.

### Study on duration of Topical AIA constituents of the extract:

The effect of apigenin glycoside and luteolin glycoside has been further investigated for the AIA, beyond the standard time of 6 hours; this activity was assessed as oedema (Table 5) and Leucocyte infiltrate inhibition (Table 6) at doses that normally produce an almost complete inhibition of oedema 6 bours after treatment.

**Table 5**

| Effect on Oedema Development | | | |
|---|---|---|---|
| Treatment | Hours after treatment | | |
| | 6 | 18 | 30 |
| Controls | 7.6±0.4 | 3.7±0.7 | 0.9±0.3 |
| Apigenin (0.037 µMol) glycoside | 0.0±0.1 | 4.5±0.4 | 0.5±0.1 |
| Luteolin (0.27µMol) glycoside | 2.0±0.5 | 2.2±0.2 | 0.8±0.2 |
| Indomethacin (1.26µMol) | 0.0±0.1 | 3.1±0.4 | 0.5±0.1 |
| Hydrocortisone (0.41 µMol) | 0.5±0.2 | 0.7±0.1 | 0.7±0.1 |
| Data in mg, means ± s.e.; p<0.05 | | | |

**Table 6**

| Effect on granulocyte infiltration (MPO activity). | | | |
|---|---|---|---|
| Treatment | Hours after treatment | | |
| | 6 | 18 | 30 |
| Controls | 18.5±2.4 | 38.3±4.8 | 22.8±7.5 |
| Apigenin (0.37µMol) glycoside | 0.2±0.1 | 8.7±0.5 | 0.9±1.4 |
| Luteolin (0.27 µMol) glycoside | 2.2±0.8 | 13.9±1.5 | 12.0±1.4 |
| Indomethacin (1.26µMol) | 0.0±0.1 | 10.0±1.6 | 6.8±3.4 |
| Hydrocortisone (0.41µMol) | 0.0±0.2 | 6.9±2.1 | 8.2±2.4 |
| One unit = 1 nMol/min. of tetraguaiacol formed at 25°C. p< 0.05 | | | |

Apigenin glycoside and indomethacin lose their antiinflammatory activity 18 hours after treatment, whereas, at that time, hydrocortisone and luteolin glycoside maintain their effect to a certain extent. The inflammation appears to naturally supress after 30 hours. The four compounds strongly and permanently inhibit the leukocyte infilteration, measured as myeloperoxidase activity.

### Percutanous absorption of Topically Active Flavonoids :

The topical AIA of apigenin glycoside, one of the main constituents of the standardized extract, appears to be more potent than Indomethacin. These findings are at variance with the common opinion that such chemical compounds are scantily absorbed through the intact skin. The percutaneous absorption of apigenin glycoside was studied in the same test conditions as used for the assessment of its AIA (inhibition of the croton oil-induced oedema in mice ears). To this purpose two doses of ³H-apigenin glycoside (60 and 6 µg/ear) were applied to the inner surface of the mice ears, with or without the usual amount of irritant (35µg/ear). The specific activity of the labelled compounds was adjusted such that constant amount of radio activity and the amount of radio activity present in the wash solutions after exhaustive washings of the ears at the given times.

The experiment shows first order absorption kinetics. The higher dose of apigenin glycoside is absorbed less rapidly (t_{½} = 4.4 h) than the lower one (t_{½} =3.4 h). the presence of corton oil enhances the absorption of the compound and the absorpotion rate becomes indentical for the both the doses (t_{½} = 2.5h).

The higher dose of apigenin glycoside almost completely inhibits the oedematous response to croton oil at least within the first 6 hours, whereas the lower dose has no effect on the development of the inflammatory response. Consequently, the enhanced absorption caused by the croton oil is not bound up with an increased permeability of the inflammed skin. The observed effect is probably due to the lipophilic properties of croton oil, which merely acts as vehicle. It can, therefore, be concluded that apigenin glycoside is absorbed through the intact skin and that its absorption-rate may be increased by a suitable pharmaceutical formulation.

### MECHANISM OF AIA OF THE EXTRACT:

Flavonoids offer the advantage of high margin of safety and lack the side effects such as ulcerogenicity over the classical antiinflammatory drugs (Agarwal, O.P., Agents Actions, 12, 298,1982; Havsteen, B., Biochem. Pharmacol., 32, 1141, 1983). Different approaches to study the antiinflammatory potential of the flavonoids have been made in recent years (Gabor, M., In "Handbook of Experimental Pharmacology: Antiinflammatory Drugs," J. R. Vane, S.H. Ferreira (Eds.) Springer, NY, 1979, p.68; Parmar, N.S and Ghosh, M.N., Ind. J. Pharmacol., 10, 277, 1978). It has been suggested that flavonoids not only increase the capillary permeability, they may also inhibit a number of stages in inflammation including granulation, tissue formation and chronic arthritis. The commonly stressed mechanism of action for antiinflammatory drugs is the inhibition of the pathways of arachidonic acid metabolism. However, while this mechanism could apply to luteolin glycoside, apigenin glycoside is unable to affect these processes (published literature) and therefore, other mechanisms of action, such as inhibition of histamine release and radical scavenging activity should be taken into account for this compound. Apigenin and luteolin also decrease leukocyte infiltration and luteolin maintained its effect even after 18 hours of treatment.

Quercetin and its glycosides have been extensively studied for their effects on inflammation and were found to control significantly the exudative and the proliferative phases of the cotton pellet granuloma (Roschin, Yu. V. and Geraschenko, G.I., Vopr. Farm. Dolnem Vostoke, 1, 134, 1973; Chem. Abstr., 83, 37683,1975; Kalshinikova, N.A and Geraschenko, G.I., Aktual. Vopr. Farm.,2, 353, 1974; Chem.Abstr., 84, 99346, 1976). Quercetin also inhibits mast cell histamine secretion (Benett, J.P., Gomperts, B.D. and Wollenweber, E., Arzneim. Forsch., 31, 433, 1981), neutrophil functions such as release of lysosomal enzymes, oxygen consumption, generation of free radicals and chemotaxis (Busse, W.W., Kipp, D.E and Middleton, E. Jr., J. Allergy Clin. Pharmacol., 73, 801, 1984). Flavonoids are known to inhibit a number of enzymes including cycloxygenase (Sekiya, S. and Okuda, H., Biochem. Biophys. Res. Commun., 105, 1090, 1982). Flavonoids present in *Euphorbia prostrata* were found to be active as oral and topical antiinflammatory agents by the inventors (Singla, A.K and Pathak, K., J. Ethanopharmacol., 27, 55, 1989; Singla, A.K. and Pathak, K., J. Ethanopharmacol., 29, 291, 1990).

### EXAMPLES

### Process of manufacture and evaluation of the standardized extract:

The plant *Euphorbia prostrata* was collected by qualified professionals from the hilly regions of Ramgarh in North India. The plant was identified and characterised by the guidelines of WHO (WHO/TRM/91.4, Programme Traditional Medicines World Health Organization Geneva, 1991) and the plant was dried under controlled conditions of temperature and humidity. The whole plant was ground to fine/coarse powder.

The powdered drug (5kg) was packed in a S.S. percolator. The extraction was affected by carrying out cold percolation with 15 It. of Ethyl Alcohol first time. 10 It. of menstruum was withdrawn and an equal volume was replaced with fresh ethyl alcohol. The process was repeated 5 times till the drug was exhausted. The alcoholic extracts were combined and concentrated under vacuum at 60°C. The concentrated extract was treated with hot water (80-90°C) and water soluble extract was obtained for the flavonoidal components. The aqueous extract was extracted with 5-10 volumes of a non polar organic solvent. The organic phasewas dehydrated with suitable desicants and concentrated under vacuum at 60°C. The concentrated extract was dried completely for several hours at 60°C under vacuum. The purified powdered extract was standardised before proceeding further.

The extract of *Euphorbia prostrata* was characterised by High Performance Liquid Chromatography (HPLC). The HPLC was performed under following conditions and using Waters system equipped with M510 pumps and data station with Millenium software.

| | |
|---|---|
| Mobile phase | Acetonitrile : 2% acetic acid (17:23) |
| Column | C₁₈ (250X4mm/5µ) |
| Flow Rate | 1 ml/min |
| Detector | UV absorbance at 350 nm |

The HPLC graph showed five peaks corresponding to the flavonoid components luteolin, 6-methoxy-quercetin glycoside, quercetin, luteolin glycoside and apigenin glycoside. Out of this the major peaks corresponded to luteolin and quercetin (these were confirmed by comparison with authentic luteolin and quercetin samples. apigenin glycoside and luteolin glycoside were used as the chemical and pharmacological marker for standardisation of the product.

### Method of preparation of capsules :

Based on the pharmacological studies it was established that each capsule should contain 15-50 mg of Euphorbia extract with HPLC finger printing compounds. Apigenin-7-glycoside 35% luteolin-7-glycoside 9 %, rest 6 % and total flavonoid content 50 %.

Hence, the Euphorbia extract used for each batch was characterised and quantified with respect to flavonoid contents as above. Based on the exact values obtained, the extract quantity of Euphorbia extract per capsules was derived. A typical example of the formula based on average values of flavonoids is as follows:

### Example 1:

| | For 1 capsule | For 10,000 capsules |
|---|---|---|
| Extract | 15 mg | 0.15 Kg |
| Lactose IP/USP | 250 mg | 2.50 Kg |
| Colloidal Silicone dioxide | 10 mg | 0.10 Kg |
| P.talc | 25 mg | 0.25 Kg |

### Example 2 :

| | For 1 capsule | For 10,000 capsules |
|---|---|---|
| Extract | 15 mg | 0.15 Kg |
| Dibasic Calcium Phosphate | 100 mg | 1.00 Kg |
| Microcrystalline Cellulose | 120 mg | 1.20 Kg |
| Starch (maize) | 60 mg | 0.60 Kg |
| Magnesium stearate | 5 mg | 0.05 Kg |

### Example 3 :

| | For 1 capsule | For 10,000 capsules |
|---|---|---|
| Extract | 15 mg | 0.15 Kg |
| Microcrystalline cellulose | 111 mg | 1.11 Kg |
| Colloidal Silicone dioxide | 3 mg | 0.03 Kg |
| Purified talc | 3 mg | 0.03 Kg |
| Magnesium stearate | 3 mg | 0.03 Kg |

The standardized extract is dissolved in Ethyl Alcohol. Lactose, Colloidal Silicone Dioxide and P. Talc are passed through fine sieves individually. The solution of standardized extract in Ethyl Alcohol is adsorbed on lactose and mixed well to achieve uniformity. The material is dried at temperature of 60-70°C till completely dried. The colloidal silicone dioxide and P.Talc are blended as lubricants. The blend is then analysed for flavonoid contents by HPLC. Based on the assay values the powder is filled in empty hard gelatin capsules at an average fill weight of 300 mg ± 10 mg. The filled capsules are sealed in air-tight unit packages after Q.C. testing.

### Method of preparation of ointment:

The standardized extract used for preparation of ointment was characterised in a similar manner as described in the preparation of capsules. Based on the exact values obtained, the exact percentage of standardized extract in ointment was derived. A typical example of the formula based on average values of flavonoids is as follows.

| | |
|---|---|
| Extract | 1% |
| Cetyl alcohol | 20 % |
| Beeswax | 24 % |
| Glycerin | 12 % |
| Sodium lauryl sulphate | 1 % |
| Propyl paraben | 0.01 % |
| Methyl paraben | 0.03 % |
| Purified water | q.s 100% |

### Example 5:

### Hydrophilic Ointment

| | |
|---|---|
| Extract | 1% |
| Methyl paraben | 0.026 % |
| Propyl paraben | 0.015 % |
| Sodium lauryl sulphate | 1% |
| Propylene glycol | 12% |
| Stearyl alcohol | 25% |
| White petrolatum | 25% |
| Purified water | q.s 100% |

### Example 6:

### Polyethylene Glycol Ointment

| | |
|---|---|
| Extract | 3% |
| Polyethylene glycol 3350 | 40% |
| Polyethylene glycol 400 | 57% |

The standardized extract is dissolved in glycerin and a small portion of purified water. Sodium lauryl sulphate, methyl paraben and propyl paraben are incorporated in the solution. The solution is warmed to 65-75°C. Simultaneously, cetyl alcohol and beeswax are molten at about 75°C. The aqueous phase is added slowly to the oily phase under constant stirring and keeping the temperature between 65°C to 75°C. The resultant emulsion is stirred further for 1 hour and the weight is made upto 100% with warm purified water, if required. The ointment is allowed to cool and de-aerated. The final product is subjected to quality control testing and filled into lacquered aluminium collapsible tubes and sealed.

### EVALUATION OF CLINICAL EFFICACY OF CAPSULES AND OINTMENT

The clinical evaluation of the pharmaceutical composition of this invention was done by University Institute of Pharmaceutical Sciences with the help of the University Health Centre, Punjab University, Chandigarh. After assessing the antiinflammatory action and toxicity testing in animals and based on the safe use of plant in Indian Traditional Medicine for other conditions, the drug was administered in oral dosage form and topical application to patients with complaints of hemorrhoids and fissures. Majority of the patients either did not respond or had relapse with other forms of therapy. Patients were normally given one/two capsules a day for a period of 3-9 days. Some patients received both oral and topical preparation. Approximately 32,000 patients had been administered the drug. Most of the patients responded to one course of treatment without any side effects. 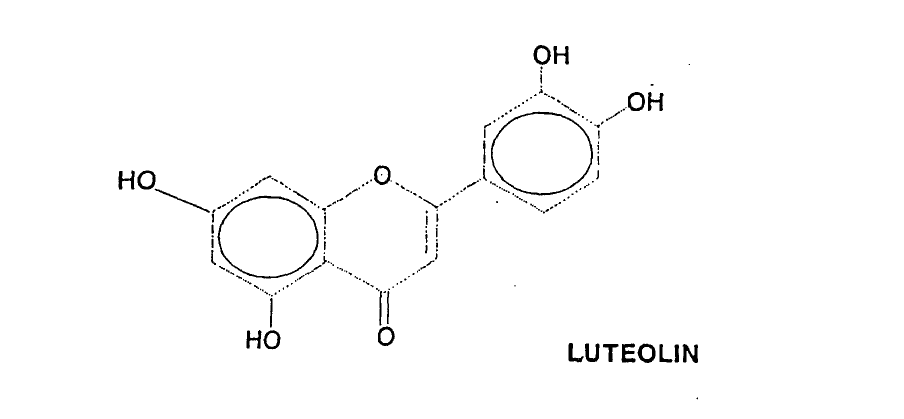 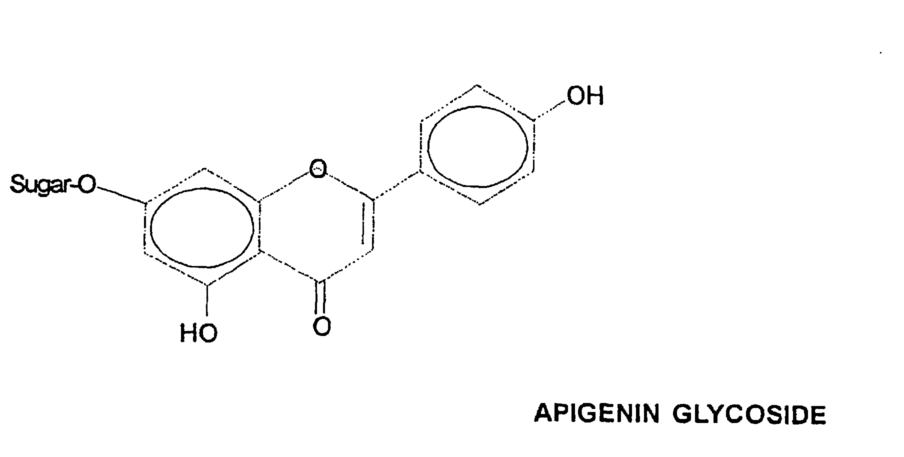 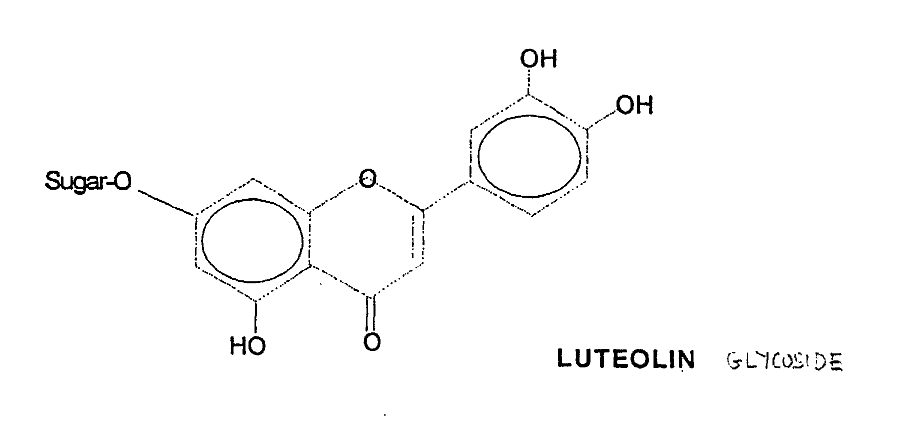 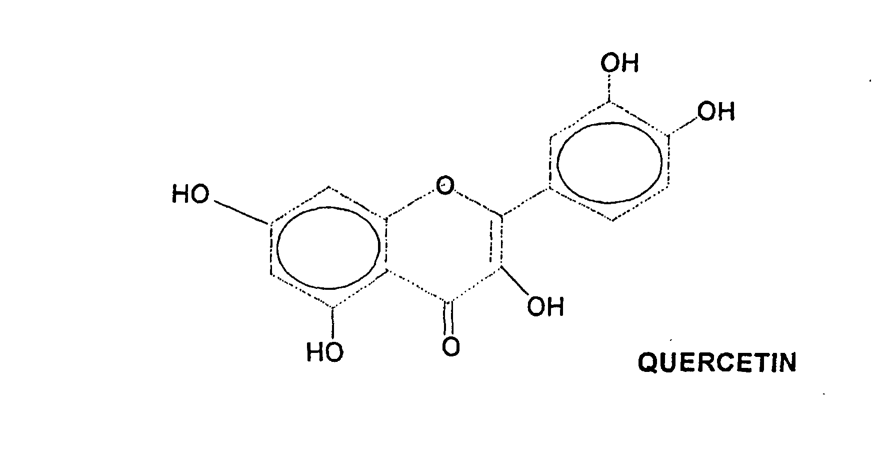 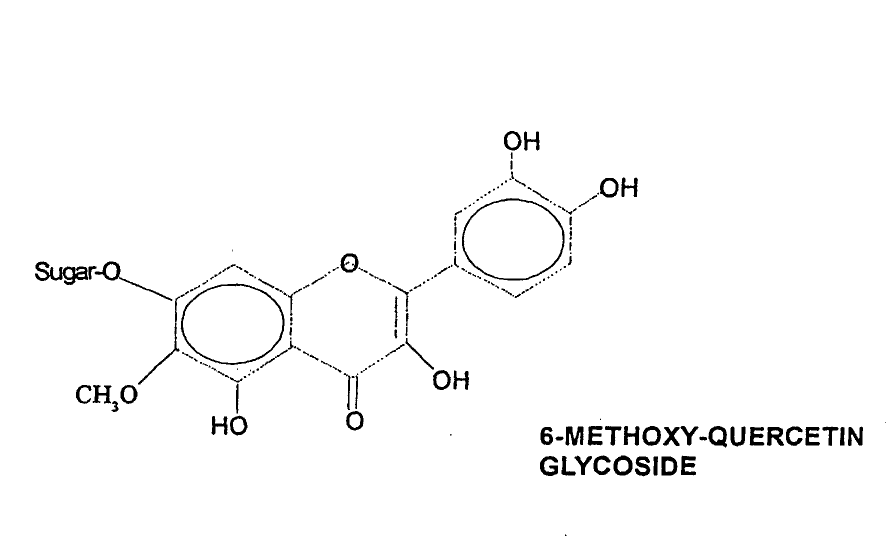 Fig. 1: Chemical Structures of five flavonoid compounds of the standardised extract of *Euphorbia prostrata*

## Claims

1. Use of a flavonoid-containing extract of the plant *Euphorbia prostrata* in the manufacture of a composition for the treatment of an anorectic or colonic disease.

2. Use of an extract according to claim 1 wherein the extract comprises apigenin-7-glycoside, luteolin-7-glycoside, 6-methoxy-quercetin-3-glycoside, quercetin and luteolin.

3. Use of an extract according to claim 1 or 2 wherein the extract comprises 30-45% by weight apigenin-7-glycoside, 3-9% by weight luteolin-7-glycoside, 1-6% by weight 6-methoxy-quercetin glycoside and 1-2% by weight quercetin and luteolin.

4. Use of an extract according to any one of claims 1 to 3 wherein the extract additionally contains tannins (5%), resins and gums (10-15%), pigments, sterols and triterpenoids.

5. Use of a composition obtained from Euphoria prostata comprising apigenin-7-glycoside, luteolin-7-glycoside, 6-methoxy-quercetin-3-glycoside, quercetin and luteolin in the manufacture of a composition for the treatment of an anorectal or colonic disease.

6. A use according to any preceding claim, wherein said anorectal disease is haemorrhoids, fissures, cracks, fistulas, abscesses, or inflammatory bowel disease such as, ulcerative colitis or Crohn's disease.

7. A use according to any preceding claim wherein the composition comprises a pharmaceutically acceptable base.

8. A use according to any preceding claim wherein the composition comprises further therapeutic agents.

9. A use according to claim 8 wherein said therapeutic agents are selected from astringents, anesthetics and/or their salts, vasoconstrictors, protectants, counterirritants, keratolytics, anti-cholinergics, wound healing agents and anti-microbial agents.

10. A use according to claim 9, wherein said therapeutic agents are astringents.

11. A use according to claim 10, wherein said astringents are selected from calamine, zinc oxide, hamamelis water, bismuthresorcinol compound, bismuth subgallate, Peruvian balsam, aluminium chlorhydroxy allantoinate and tannic acid.

12. A use according to claim 10 wherein the amount of said astringents vary between 0.2% and 60% by weight.

13. A use according to claim 9, wherein said therapeutic agents are anesthetics and/or their salts.

14. A use according to claim 13, wherein said anesthetics and/or their salts are selected from benzocaine, diperomon, pramoxine. camphor, dibucaine, phenol, tetracaine, and phenacaine.

15. A use according to claim 13 wherein the amount of said anesthetics vary between 0.25% and 25% by weight.

16. A use according to claim 9, wherein said therapeutic agents are vasoconstrictors.

17. A use according to claim 16, wherein said vasoconstrictors are selected from ephedrine, phenylephrine, phenylephrine and/or their salts.

18. A use according to claim 16 wherein the amount of said vasoconstrictors may vary between 0.005% and 1.5% by weight.

19. A use according to claim 9, wherein said therapeutic agents are counterirritants.

20. A use according to claim 19, wherein said counterirritant is menthol and is present in an amount between 0.25 and 2.5% by weight.

21. A use as claimed in claim 9, wherein said therapeutic agents are protectants.

22. A use as claimed in claim 21, wherein said protectants are selected from aluminium hydroxide gel, calamine, cocoa butter, cod or shark liver oil, starch, white petrolatum, wool alcohol, zinc oxide, vegetable or castor oil, polyethylene glycol, propylene glycol.

23. A use according to claim 21 wherein said protectants are present in an amount between 5.0% and 88.0% by weight.

24. A use according to claim 9, wherein said therapeutic agents are wound healing agents.

25. A use according to claim 24, wherein said wound healing agents are selected from vitamin A, vitamin D, Peruvian balsam, cod liver oil and live yeast cell derivatives.

26. A use according to claim 24 wherein said vitamin A and vitamin D are present in an amount between 0.005% to 0.04% by weight, said Peruvian balsam is present in amount between 0.5% to 2.5%, cod liver oil is present in an amount between 1.0% to 6.0% and live yeast cell derivatives are present in an amount between 2 - 50,000 units per gram.

27. A use according to claim 9, wherein said therapeutic agents are antimicrobial agents.

28. A use according to claim 27, wherein said antimicrobial agents are selected from benzethonium chloride, benzalkonium chloride, boric acid, 8-quinolinol benzoate, secondary amyltricresols, cetylpyridiniium chloride, phenol, menthol, chlorothymol, camphor and 8-hydroxyquinoline sulfate.

29. A use according to claim 27, wherein said antimicrobial agents are present in an amount between 0.02% to 40% by weight.

30. A use according to claimed in claim 9, wherein said therapeutic agents are keratolytics.

31. A use according to claim 30, wherein said keratolytics are selected from aluminium chlorohydroxy allanoinate and resorcinol.

32. A use according to claim 30 wherein said keratolytics are present in an amount between 0.2% and 3.5% by weight.

33. A use according to claim 9, wherein said therapeutic agents are anti-cholinergics.

34. A use according to claim 33, wherein said anticholinergics are selected from atropine or other solanaceous type alkaloids, either alone or in combination.

35. A use according to claim 33 wherein the amount of said anti-cholinergics vary between 0.02% and 0.1 % by weight.

36. A use according to any preceding claim wherein the composition is in the form of a local cream, ointment, solution, spray, foam, suppository, medicated pad, bandage, powder, suspension, film, flake, oral hard gelatine capsules, soft gelatine capsules, tablets (coated and uncoated), sustained release dosage form, liquid, lozenges, buccal or any modified delivery dosage form, waffers, caplets, parenteral dosage to be infiltered at the site of the injection.

37. A process for the manufacture of a an extract for use according to claim 1 which comprises drying the plant *Euphorbia Prostrata* under controlled conditions of temperature and humidity, making a powder from the said dried plant, extracting the dry coarse powder to form an extract, filtering and distilling the said extract repetitively, dehydrating the said extract, drying the extract in a vacuum to produce the desired pharmaceutical extract capable of being used along with a suitable carrier/base.

38. A process as claimed in claim 37, wherein the said dry coarse powder is extracted with aqueous alcohol.

39. A process as claimed in claim 37, wherein the extract is filtered and extracted with ethyl acetate.

40. A process as claimed in claim 37, wherein the dehydration is carried out over anhydrous sodium sulphate.

## Patentansprüche

1. Verwendung eines Flavonoide enthaltenden Extraktes aus der Pflanze *Euphorbia prostrata* zur Herstellung einer Zusammensetzung für die Behandlung von Anorektal- und Kolonerkrankungen.

2. Verwendung eines Extraktes nach Anspruch 1, wobei der Extrakt Apigenin-7-glykosid, Luteolin-7-glykosid, 6-Methoxy-quercetin-3-glykosid, Quercitin und Luteolin umfaßt.

3. Verwendung eines Extraktes nach Anspruch 1 oder 2, wobei der Extrakt 30-45 Gew.-% Apigenin-7-glykosid, 3-9 Gew.-% Luteolin-7-glykosid, 1-6 Gew.-% 6-Methoxyquercetinglykosid und 1-2 Gew.-% Quercetin und Luteolin umfaßt.

4. Verwendung eines Extraktes nach einem der Ansprüche 1 bis 3, wobei der Extrakt zusätzlich Gerbstoffe (5%), Harze und Gummis (10-15%), Pigmente, Sterole und Triterpene enthält.

5. Verwendung einer Zusammensetzung, erhalten aus *Euphorbia prostrata,* umfassend Apigenin-7-glykosid, Luteolin-7-glykosid, 6-Methoxyquercetin-3-glykosid, Quercetin und Luteolin, zur Herstellung einer Zusammensetzung für die Behandlung einer Anorektal- oder Kolonerkrankung.

6. Verwendung nach einem vorherigen Anspruch, wobei die Anorektalerkrankung Hämorrhoiden, Fissuren, Risse, Fisteln, Abszesse oder entzündliche Darmerkrankungen, wie Colitis Ulcerosa oder Morbus Crohn, ist.

7. Verwendung nach einem vorherigen Anspruch, wobei die Zusammensetzung einen pharmazeutisch verträglichen Grundbestandteil umfaßt.

8. Verwendung nach einem vorherigen Anspruch, wobei die Zusammensetzung ferner therapeutische Mittel umfaßt.

9. Verwendung nach Anspruch 8, wobei die therapeutischen Mittel aus Adstringenzien, Anästhetikas und/oder ihren Salzen, Vasokonstriktoren, Schutzmitteln, Gegenreizmitteln, Keratolytikas, Anti-Cholinergikas, Wundheilungsmitteln und anti-mikrobiellen Mitteln ausgewählt sind.

10. Verwendung nach Anspruch 9, wobei die therapeutischen Mittel Astringenzien sind.

11. Verwendung nach Anspruch 10, wobei die Astringenzien aus Kalamin, Zinkoxid, Hamameliswasser, Bismuthresorcinol-Verbindung, Bismuthsubgallat, Perubalsam, Aluminiumchlorhydroxyallantoinat und Gerbsäuren ausgewählt sind.

12. Verwendung nach Anspruch 10, wobei die Menge der Astrigenzien zwischen 0,2 und 60 Gew.-% variiert.

13. Verwendung nach Anspruch 9, wobei die therapeutischen Mittel Anästhetikas und/oder ihre Salze sind.

14. Verwendung nach Anspruch 13, wobei die Anästhetikas und/ihre Salze aus Benzocain, Diperomon, Pramoxin, Kampfer, Dibucain, Phenol, Tetracain und Phenacain ausgewählt sind.

15. Verwendung nach Anspruch 13, wobei die Menge der Anästhetika zwischen 0,25 und 25 Gew.-% variiert.

16. Verwendung nach Anspruch 9, wobei die therapeutischen Mittel Vasokonstriktoren sind.

17. Verwendung nach Anspruch 16, wobei die Vasokonstriktoren aus Ephedrin, Phenylephrin, Phenylephrine und/oder ihren Salzen ausgewählt sind.

18. Verwendung nach Anspruch 16, wobei die Menge der Vasokonstriktoren zwischen 0,005 und 1,5 Gew.-% variieren kann.

19. Verwendung nach Anspruch 9, wobei die therapeutischen Mittel Gegenreizmittel sind.

20. Verwendung nach Anspruch 19, wobei das Gegenreizmittel Menthol ist und in einer Menge zwischen 0,25 und 2,5 Gew.-% vorliegt.

21. Verwendung nach Anspruch 9, wobei die therapeutischen Mittel Schutzmittel sind.

22. Verwendung nach Anspruch 21, wobei die Schutzmittel aus Aluminiumhydroxidgel, Kalamin, Kakaobutter, Dorsch- oder Haifischlebertran, Stärke, Alvolen, Wollfettalkohol, Zinkoxid, Pflanzenöl oder Rizinusöl, Polyethylenglykol, Polypropylenglykol ausgewählt sind.

23. Verwendung nach Anspruch 21, wobei die Schutzmittel in einer Menge zwischen 5,0 und 88,0 Gew.-% vorliegen.

24. Verwendung nach Anspruch 9, wobei die therapeutischen Mittel Wundheilungsmittel sind.

25. Verwendung nach Anspruch 24, wobei die Wundheilungsmittel aus Vitamin A, Vitamin D, Perubalsam, Dorschlebertran oder lebenden Hefezellderivaten ausgewählt sind.

26. Verwendung nach Anspruch 24, wobei das Vitamin A und Vitamin D in einer Menge zwischen 0,005 bis 0,04 Gew.-% vorliegen, der Perubalsam in einer Menge zwischen 0,5% bis 2,5% vorliegt, der Dorschlebertran in einer Menge zwischen 1,0% bis 6,0% vorliegt und die lebenden Hefezellderivate in einer Menge zwischen 2 - 50.000 Units pro Gramm vorliegen.

27. Verwendung nach Anspruch 9, wobei die therapeutischen Mittel antimikrobielle Mittel sind.

28. Verwendung nach Anspruch 27, wobei die anti-mikrobiellen Mittel aus Benzethoniumchlorid, Benzalkoniumchlorid, Borsäure, 8-Chinolinolbenzoat, sekundären Amyltrikresolen, Cetylpyridiniumchlorid, Phenol, Menthol, Chlorthymol, Kampfer und 8-Hydroxychinolinsulfat ausgewählt sind.

29. Verwendung nach Anspruch 27, wobei die anti-mikrobiellen Mittel in einer Menge zwischen 0,02 bis 40 Gew.-% vorliegen.

30. Verwendung nach Anspruch 9, wobei die therapeutischen Mittel Keratolytikas sind.

31. Verwendung nach Anspruch 30, wobei die Keratolytikas aus Aluminiumchlorhydroxyallanoinat und Resorcinol ausgewählt sind.

32. Verwendung nach Anspruch 30, wobei die Keratolytikas in einer Menge zwischen 0,2 und 3,5 Gew.-% vorliegen.

33. Verwendung nach Anspruch 9, wobei die therapeutischen Mittel Anti-Cholinergikas sind.

34. Verwendung nach Anspruch 33, wobei die Anti-Cholinergikas aus Atropin oder anderen Alkaloiden des Solanumtyps, entweder allein oder in Kombination, ausgewählt sind.

35. Verwendung nach Anspruch 33, wobei die Menge der Anti-Cholinergikas zwischen 0,02 und 0,1 Gew.-% variiert.

36. Verwendung nach einem vorhergehenden Anspruch, wobei die Zusammensetzung in Form einer lokalen Creme, Salbe, Lösung, Spray, Schaum, Zäpfchen, medizinischen Kompresse, Verband, Puder, Suspension, Film, Flocke, oralen Hartgelatinekapseln, weichen Gelatinekapseln, Tabletten (beschichtet und unbeschichtet), Dauerfreisetzungsverabreichungsform, Flüssigkeit, Pastillen, Speichel- ' oder anderen modifizierten Verteilungsverabreichungsformen, Waffeln, Caplets, parenteralen Dosierung zum Einfiltrieren an der Injektionsstelle vorliegt.

37. Verfahren zur Herstellung eines Extraktes zur Verwendung nach Anspruch 1, welches das Trocknen der Pflanze *Euphorbia prostrata* unter kontrollierten Temperatur- und Feuchtigkeitsbedingungen, das Herstellen eines Pulvers aus der getrockneten Pflanze, das Extrahieren des trockenen groben Pulvers zur Bildung eines Extraktes, das wiederholte Filtrieren und Destillieren des Extraktes, das Dehydratisieren des Extraktes, und das Trocknen des Extraktes in einem Vakuum, um den gewünschten pharmazeutischen Extrakt herzustellen, welcher befähigt ist, zusammen mit einem verträglichen Träger/Grundbestandteil verwendet zu werden, umfaßt.

38. Verfahren nach Anspruch 37, wobei das trockene grobe Pulver mit wäßrigem Alkohol extrahiert wird.

39. Verfahren nach Anspruch 37, wobei der Extrakt filtriert wird und mit Ethylacetat extrahiert wird.

40. Verfahren nach Anspruch 37, wobei die Dehydratisierung über wasserfreiem Natriumsulfat durchgeführt wird.

## Revendications

1. Utilisation d'un extrait de la plante *Euphorbia prostrata* contenant des flavonoïdes dans la fabrication d'une composition destinée au traitement d'une maladie ano-rectale ou du côlon.

2. Utilisation d'un extrait selon la revendication 1, dans laquelle l'extrait comprend de l'apigénine-7-glycoside, du lutéoline-7-glycoside, du 6-méthoxyquercétine-3-glycoside, de la quercétine et de la lutéoline.

3. Utilisation d'un extrait selon la revendication 1 ou 2, dans laquelle l'extrait comprend 30-45% en poids d'apigénine-7-glycoside, 3-9% en poids de lutéoline-7-glycoside, 1-6% en poids de 6-méthoxyquercétineglycoside et 1-2% en poids de quercétine et de lutéoline.

4. Utilisation d'un extrait selon l'une quelconque des revendications 1 à 3, dans laquelle l'extrait contient en outre des tannins (5%), des résines et des gommes (10-15%), des pigments, des stérols et des triterpénoïdes.

5. Utilisation d'une composition obtenue à partir de *Euphorbia prostrata* comprenant de l'apigénine-7-glycoside, du lutéoline-7-glycoside, du 6-méthoxyquercétine-3-glycoside, de la quercétine et de la lutéoline dans la fabrication d'une composition destinée au traitement d'une maladie ano-rectale ou du côlon.

6. Utilisation selon l'une quelconque des revendications précédentes, dans laquelle ladite maladie ano-rectale est constituée par des hémorroïdes, des fissures, des gerçures, des fistules, des abcès, ou une maladie inflammatoire de l'intestin telle que la colite ulcéreuse ou la maladie de Crohn.

7. Utilisation selon l'une quelconque des revendications précédentes, dans laquelle la composition comprend une base acceptable sur le plan pharmaceutique.

8. Utilisation selon l'une quelconque des revendications précédentes, dans laquelle la composition comprend en outre des agents thérapeutiques.

9. Utilisation selon la revendication 8, dans laquelle lesdits agents thérapeutiques sont choisis parmi les astringents, les anesthésiques et/ou leurs sels, les vasoconstricteurs, les protecteurs, les révulsifs, les kératolytiques, les anticholinergiques, les agents de cicatrisation et les agents antimicrobiens.

10. Utilisation selon la revendication 9, dans laquelle lesdits agents thérapeutiques sont des astringents.

11. Utilisation selon la revendication 10, dans laquelle lesdits astringents sont choisis parmi la calamine, l'oxyde de zinc, l'extrait d'hamamélis, un composé de bismuthrésorcinol, le sous-gallate de bismuth, le baume du Pérou, le chlorhydroxyallantoïnate d'aluminium et l'acide tannique.

12. Utilisation selon la revendication 10, dans laquelle la quantité desdits astringents varie entre 0,2% et 60% en poids.

13. Utilisation selon la revendication 9, dans laquelle lesdits agents thérapeutiques sont des anesthésiques et/ou leurs sels.

14. Utilisation selon la revendication 13, dans laquelle lesdits anesthésiques et/ou leurs sels sont choisis parmi la benzocaïne, le dipéromon, la pramoxine, le camphre, la dibucaïne, le phénol, la tétracaïne et la phénacaïne.

15. Utilisation selon la revendication 13, dans laquelle la quantité desdits anesthésiques varie entre 0,25% et 25% en poids.

16. Utilisation selon la revendication 9, dans laquelle lesdits agents thérapeutiques sont des vasoconstricteurs.

17. Utilisation selon la revendication 16, dans laquelle lesdits vasoconstricteurs sont choisis parmi l'éphédrine, la phényléphrine et/ou leurs sels.

18. Utilisation selon la revendication 16, dans laquelle la quantité desdits vasoconstricteurs peuvent varier entre 0,005% et 1,5% en poids.

19. Utilisation selon la revendication 9, dans laquelle lesdits agents thérapeutiques sont des révulsifs.

20. Utilisation selon la revendication 19, dans laquelle ledit révulsif est le menthol et est présent en une quantité comprise entre 0,25 et 2,5% en poids.

21. Utilisation telle que revendiquée dans la revendication 9, dans laquelle lesdits agents thérapeutiques sont des protecteurs.

22. Utilisation telle que revendiquée dans la revendication 21, dans laquelle lesdits protecteurs sont choisis parmi le gel d'hydroxyde d'aluminium, la calamine, le beurre de cacao, l'huile de foie de morue ou de requin, l'amidon, la vaseline blanche, l'alcool de laine, l'oxyde de zinc, l'huile végétale ou de ricin, le polyéthylèneglycol, le propylèneglycol.

23. Utilisation selon la revendication 21, dans laquelle lesdits protecteurs sont présents en une quantité comprise entre 5,0% et 88,0% en poids.

24. Utilisation selon la revendication 9, dans laquelle lesdits agents thérapeutiques sont des agents de cicatrisation.

25. Utilisation selon la revendication 24, dans laquelle lesdits agents de cicatrisation sont choisis parmi la vitamine A, la vitamine D, le baume du Pérou, l'huile de foie de morue et des dérivés de cellules de levures vivantes.

26. Utilisation selon la revendication 24, dans laquelle ladite vitamine A et ladite vitamine D sont présentes en une quantité comprise entre 0,005% et 0,04% en poids, ledit baume du Pérou est présent en une quantité comprise entre 0,5% et 2,5%, l'huile de foie de morue est présente en une quantité comprise entre 1,0% et 6,0% et les dérivés de cellules de levures vivantes sont présents en une quantité comprise entre 2 et 50 000 unités par gramme.

27. Utilisation selon la revendication 9, dans laquelle lesdits agents thérapeutiques sont des agents antimicrobiens.

28. Utilisation selon la revendication 27, dans laquelle lesdits agents antimicrobiens sont choisis parmi le chlorure de benzéthonium, le chlorure de benzalkonium, l'acide borique, le benzoate de 8-quinoléinol, les amyltricrésols secondaires, le chlorure de cétylpyridinium, le phénol, le menthol, le chlorothymol, le camphre et le sulfate de 8-hydroxyquinoléine.

29. Utilisation selon la revendication 27, dans laquelle lesdits agents antimicrobiens sont présents en une quantité comprise entre 0,02% et 40% en poids.

30. Utilisation selon la revendication 9, dans laquelle lesdits agents thérapeutiques sont des kératolytiques.

31. Utilisation selon la revendication 30, dans laquelle lesdits kératolytiques sont choisis parmi le chlorhydroxyallantoïnate d'aluminium et le résorcinol.

32. Utilisation selon la revendication 30, dans laquelle lesdits kératolytiques sont présents en une quantité comprise entre 0,2% et 3,5% en poids.

33. Utilisation selon la revendication 9, dans laquelle lesdits agents thérapeutiques sont des anticholinergiques.

34. Utilisation selon la revendication 33, dans laquelle lesdits anticholinergiques sont choisis parmi l'atropine et d'autres types d'alcaloïdes de solanacées, soit seuls soit en association.

35. Utilisation selon la revendication 33, dans laquelle la quantité desdits anticholinergiques varie entre 0,02% et 0,1% en poids.

36. Utilisation selon l'une quelconque des revendications précédentes, dans laquelle la composition se présente sous forme d'une crème à usage local, d'une pommade, d'une solution, d'une pulvérisation, d'une mousse, d'un suppositoire, d'un tampon médicamenteux, d'un bandage, d'une poudre, d'une suspension, d'un film, de flocons, de capsules de gélatine dures orales, de capsules de gélatine molles, de comprimés (enrobés ou non enrobés), d'une forme posologique à libération prolongée, de liquide, de pastilles, d'une forme posologique gingivo-jugale ou de toute forme posologique à délivrance modifiée, de cachets, de comprimés, de préparation parentérale pour infiltration au point d'injection.

37. Procédé de fabrication d'un extrait destiné à l'utilisation selon la revendication 1, qui comprend le séchage de la plante *Euphorbia Prostrata* dans des conditions régulées de température et d'humidité, la préparation d'une poudre à partir de ladite plante séchée, l'extraction de la poudre grossière sèche pour former un extrait, la filtration et la distillation dudit extrait de manière répétée, la déshydratation dudit extrait, le séchage de l'extrait sous vide pour produire l'extrait pharmaceutique désiré utilisable avec un véhicule/une base approprié(e).

38. Procédé selon la revendication 37, dans lequel ladite poudre grossière sèche est extraite avec de l'alcool aqueux.

39. Procédé selon la revendication 37, dans lequel l'extrait est filtré et extrait avec de l'acétate d'éthyle.

40. Procédé selon la revendication 37, dans lequel la déshydratation est réalisée sur du sulfate de sodium anhydre.
